# EUROPÄISCHE PATENTSCHRIFT

(11) **EP 1 591 105 B1**
(45) Veröffentlichungstag und Bekanntmachung des Hinweises auf die Patenterteilung: **27.05.2020**
(21) Anmeldenummer: 05005536.7
(22) Anmeldetag: 15.03.2005
(51) Int. Cl.: A61K 8/31, A61K 8/34, A61K 8/36, A61K 8/44, A61K 8/49, A61K 8/67, A61Q 17/04, A61K 8/97, A61K 8/35, A61K 8/9789, A61K 8/9794, A61K 8/365

(54) **Verwendung von Antioxidantien zur Herstellung einer pharmazeutischen oder kosmetischen Zusammensetzung zum Schutz der Haut gegen Schädigung durch Infrarot-Strahlung**
Use of antioxidants for the preparation of pharmaceutical or cosmetic compositions for protecting the skin from damages by infrared-radiations
Utilisation d'antioxydants pour préparer des compositions pharmaceutiques ou cosmétiques pour protéger la peau contre les dommages provoqués par les rayonnements infra-rouges

(30) Priorität: 17.03.2004 EP 04006336
(43) Veröffentlichungstag der Anmeldung: 02.11.2005
(60) Teilanmeldung: 09012941.2
(73) Patentinhaber: STADA ARZNEIMITTEL AG, 61118 Bad Vilbel (DE)
(72) Erfinder:
(74) Vertreter: Hamm&Wittkopp Patentanwälte PartmbB

(56) Entgegenhaltungen:
- EP-A- 1 120 407
- WO-A-02/081027
- FR-A- 2 802 421
- US-A- 6 159 481
- US-A1- 2003 060 426
- US-A1- 2003 091 518
- "ARCHIVES OF DERMATOLOGICAL RESEARCH" ARCHIVES OF DERMATOLOGICAL RESEARCH, SPRINGER, INTERNATIONAL, BERLIN, DE, Bd. 295, Nr. 6-8, Februar 2004 (2004-02), Seite 367, XP002291087 ISSN: 0340-3696
- PATENT ABSTRACTS OF JAPAN Bd. 2002, Nr. 09, 4. September 2002 (2002-09-04) & JP 2002 138027 A (KOSE CORP; PENTAPHARM JAPAN KK), 14. Mai 2002 (2002-05-14)
- DATABASE WPI Section Ch, Week 200312 Derwent Publications Ltd., London, GB; Class B07, AN 2003-123223 XP002327458 & JP 2002 249772 A (OFFICE KUWAJIMA YG) 6. September 2002 (2002-09-06)

## Beschreibung

Die Erfindung betrifft die Verwendung von Antioxidantien zur Herstellung einer pharmazeutischen oder kosmetischen Zusammensetzung zum Schutz der Haut gegen Schädigung durch Infrarot-Strahlung.

Die schädigende Wirkung des ultravioletten Teils der Sonnenstrahlung auf die Haut ist seit langem bekannt. Zum Schutz der Haut sowohl vor UVA-Strahlung als auch vor UVB-Strahlung sind zahlreiche chemische und physikalische Filtersubstanzen entwickelt worden, die durch Auftragen auf die Haut in Form dermatologischer oder kosmetischer Zusammensetzungen die Wirkung der Sonnenstrahlen im UVA- und UVB-Bereich abschwächen.

Neuere Untersuchungen haben ergeben, dass auch infrarote Strahlung einen schädigenden Einfluss auf die Haut hat. Infrarotstrahlung ist nicht nur wesentlicher Bestandteil des Sonnenlichts, sondern auch des künstlichen Lichts.

Nahes Infrarotlicht, IR-A-Licht, mit einem Wellenlängenbereich von 760-1400 nm, aktiviert die Bildung von Matrixmetalloproteinase-1 in den Fibroblasten der menschlichen Haut, vermittelt über Aktivierung des MAPK-Signalwegs. Metrixmetalloptoeinase-1 ist für die vorzeitige Hautalterung mitverantwortlich.

Der photobiologische Mechanismus, über den das IR-A-Licht die MAPKinase aktiviert, ist unbekannt.

Aufgabe der vorliegenden Erfindung ist es, eine Möglichkeit zum Schutz der Haut gegen Schädigung durch IR-Strahlung anzugeben.

Diese Aufgabe wird durch die im unabhängigen Anspruch definierte Verwendung gelöst.

Die abhängigen Ansprüche sind vorteilhafte Ausführungsformen der Erfindung.

Überraschend wurde gefunden, dass die Verwendung von Antioxidantien zur Herstellung von pharmazeutischen oder kosmetischen Zusammensetzungen zu Produkten führt, die die Haut wirksam gegen den schädigenden Einfluss von IR-Strahlung schützen.

Im Stand der Technik sind zahlreiche kosmetische Zusammensetzungen bekannt, die unter anderem Antioxidantien enthalten. So betrifft beispielsweise EP-A-1 120 407 einen Inhibitor für oxidativen Stress, der die Expression zytotoxischer Proteine und die Aktivierung eines Gentranskriptionsregulationsfaktors unterdrückt und Cystein bzw. Cysteinderivate als Wirkstoff enthält. Die Zusammensetzungen gemäß EP-A-1 120 407 können unter anderem Antioxidantien enthalten. Die US 6,159,481 ist auf eine Sonnenschutz- und antioxidative Zubereitung gerichtet, die einen Selenhefekomplex umfasst. Auch die Zusammensetzungen gemäß US 6,159,481 können des Weiteren Antioxidantien enthalten. Die US 2003/0091518 betrifft kosmetische und/oder pharmazeutische Zusammensetzungen, umfassend Brassicaceae-Extrakt und eine oder mehrere Komponenten aus der Gruppe, bestehend aus unter anderem Antioxidantien und UV/IR-Schutzfaktoren, sowie deren Verwendung als Haut- und Haarpflegemittel. FR 2 802 421 betrifft eine feuchtigkeitsspendende kosmetische Tages- und Nachtcreme, die zur Gesichts- und Handpflege von Personen entwickelt wurde, die empfindlich gegenüber Wärmeeinwirkung durch Infrarot-Strahlung reagieren, Die Zusammensetzungen gemäß FR 2 802 421 können neben den wirksamen Bestandteilen unter anderem Antioxidantien enthalten. WO 02/081027 ist auf Zusammensetzungen, umfassend Antioxidantien gerichtet, sowie ihre Verwendung als kosmetische und pharmazeutische Zusammensetzung zum Schutz der Haut, um Zell- und Hautschäden, die durch freie Sauerstoffradikale verursacht werden, vorzubeugen. Im Stand der Technik findet sich jedoch kein Hinweis darauf, dass die in den Zusammensetzungen verwendeten Antioxidantien dem Schutz vor IR-Strahlen dienen. Damit konnte der Stand der Technik nicht den Weg zur vorliegenden Erfindung weisen.

Es hat sich überraschender Weise herausgestellt, dass pharmazeutische oder kosmetische Zusammensetzungen umfassend Antioxidantien ausgewählt aus der Gruppe bestehend aus: Kaffeesäure und deren Derivate, insbesondere Ester der Kaffeesäure, Carotinoide (z.B. Lycopen, Lutein), Thiole (z.B. N-Acetylcystein), Vitis Vinifera (Grape) seed extract, Cichorium intubybus (chicory) leaf extract, Leontopodium Alpinum Extract (Edelweiss), Grünteeextrakt, Curcumin, Silymarin, Apigenin, Resveratrol oder Emblica einen wirksamen Schutz vor IR-Strahlung bieten. Sie können einzeln oder in Kombination mehrerer Antioxidantien eingesetzt werden.

Emblica wird aus den Früchten der Heilpflanze Phyllantus emblica schonend durch Wasserdampfdestillation gewonnen. Hauptinhaltsstoffe von Emblica sind die niedermolekularen Gerbsäuren Emblicanin A und B.

Unabhängig von einer wissenschaftlichen Lehre vermutet die Anmelderin, dass der wirksame IR-Schutz durch Verabreichung von Antoxidantien enthaltenden pharmazeutischen Zusammensetzungen auf einem Einfluss der Antioxdantien auf die Aktivierung des MAPK-Signalwegs beruht. Der photobiologische Mechanismus, über den das IR-Licht die MAPKinase aktiviert, ist zwar unbekannt, doch vermutet die Anmelderin, dass diese Aktivierung über reaktive Sauerstoffspezies, sogenannte freie Radikale, vermittelt wird. Die erfindungsgemäße Verwendung von Antioxidantien führt vermutlich zu einer Minderung des Gehaltes an reaktiver Sauerstoffspezies. Dadurch wird der durch IR-Strahlung aktivierten und über den MAPKinase-Signalweg vermittelten Bildung von Matrixmetalloproteinase-1 entgegengewirkt.

Die erfindungsgemäßen Zusammensetzungen können topisch, oral, parenteral oder rektal verabreicht werden.

Die topisch zu verabreichenden Zusammensetzungen werden bevorzugt in Form von Creme, Salbe, Paste, Lotion, Gel, Hydrodispersionsgel, Aerosol, Spray, oder Stift auf die Haut aufgetragen.

Als bevorzugte Antoxidantien für die topische Anwendung haben sich insbesondere Emblica, Lutein, Lycopen sowie Grünteeextrakt, Curcumin, Silymarin, Apigenin und Resveratrol erwiesen.

Der Gehalt an Antioxidantien in den topisch anzuwendenden pharmazeutischen Zusammensetzungen beträgt 0,001-25 Gew.-%, insbesondere 0,01-10 Gew.-%.

Topisch zu verabreichende Zusammensetzungen können weitere übliche Zusatzstoffe für dermatologische Zusammensetzungen, wie z.B. Verdickungsmittel, Glättungsmittel, anfeuchtende und/oder feuchthaltende Substanzen, oberflächenaktive Mittel, Konservierungsmittel, Entschäumer, Fette, Öle, Wachse, Konservierungsmittel, Bakterizide, Parfüme, Treibmittel, Farbstoffe und/oder Pigmente oder dergleichen umfassen.

Als Verdickungsmittel werden, sofern enthalten, bevorzugt Xanthan oder Polyacrylsäuren und deren Derivate, wie beispielsweise die aus der Carbopol-Reihe oder auch Pemulen TR-1 eingesetzt.

Derartige Zusammensetzungen werden bevorzugt vor, während oder nach einer Bestrahlung der Haut mit IR-Strahlen angewendet.

Die topisch zu verabreichenden Zusammensetzungen umfassen in bevorzugten Ausführungsformen der Erfindung des weiteren einen oder mehrere anorganische und/oder organische UV-Filter.

Derartige Zusammensetzungen können bevorzugt während der Bestrahlung der Haut mit Sonnenstrahlen zum Schutz vor Schäden durch IR- und UV-Strahlen angewendet werden.

Vorteilhafte anorganische UV-Filter sind Metalloxide, insbesondere Oxide des Titans oder Zinks. Wenn anorganische Pigmente enthalten sind, werden diese vorzugsweise in mikronisierter Form eingesetzt, vorzugsweise in Teilchengrößen kleiner 100 µm. Sie sind dann bevorzugt in Mengen von 1-25 Gew.-%, insbesondere 1-10 Gew.-% enthalten. Dabei ist es besonders vorteilhaft, wenn die physikalischen Lichtschutzfilter in hydrophober Form vorliegen, dass heißt, dass sie oberflächig wasserabweisend ausgestaltet sind. Dies kann z.B. dadurch erfolgen, dass die Pigmentteilchen mit einer hydrophoben Oberflächenschicht aus Silikon überzogen werden. Derartige Pigmente sind kommerziell erhältlich beispielsweise von der Firm Tayca unter der Handelsbezeichnung MT100T.

Erfindungsgemäße organische UV-Filter sind öllösliche oder wasserlösliche UV-A- und/oder UV-B-Filter.

Als geeignete öllösliche UV-Filter sind zu nennen:
- Ester der Zimtsäure, beispielsweise P-Methoxyzimtsäureisoamylester (Neo Heliopan 1000) oder Methoxyzimtsäureethylhexylester (Neo Heliopan AV);
- P-Aminobenzoësäure, ihre Ester und Derivate, beispielsweise 2-Ethylhexyl-p-dimethylaminobenzoat (Eskalol 507);
- Benzophenone, beispielsweise Benzophenon-3 (Neo Heliopan BB);
- Benzylidencampherderivate, beispielsweise 4-Methylbenzylidencampher (Eusolex 6300);
- Hydroxyphenyltriazine, beispielsweise Bis-ethylhexyloxyphenolmethoxyphenyl (Tinosorb S);
- Dibenzoylmethane, beispielsweise Butylmethoxydibenzoylmethan (Parsol 1789);
- Octyltriazon (Uvinul T150) und
- Octocrylen (Neo Heliopan 303).

Öllösliche UV-Filter sind, sofern enthalten, bevorzugt in Mengen von 0,1-15 Gew.-%, vorzugsweise 0,5-10 Gew.-%, insbesondere 0,5-8 Gew.-%, in den topisch zu verabreichenden Zusammensetzungen enthalten.

Als geeignete wasserlösliche UV-Filter sind zu nennen:
- Salze der 2-Phenylbenzimidazol-5-sulfonsäure (Neo Heliopan Hydro), sowie die Sulfonsäure selbst;
- 2,2'-(1,4-Phenylen)bis(1H-benzimidazol-4,6-disulfonsäure Mononatriumsalz) (Neo Heliopan AP)
- Sulfonsäure-Derivate des 3-Benzylidencamphers, beispielsweise 4-(2-oxo-3-bornylidenmethyl)benzolsulfonsäure, 2-Methyl-5-(2-oxo-3-bornylidenmethyl)sulfonsäure und ihre Salze.

Auch die wasserlöslichen UV-Filter sind, sofern enthalten, bevorzugt in Mengen von 0,1-15 Gew.-%, vorzugsweise 0,5-10 Gew.-%, insbesondere 0,5-8 Gew.-%, in den topisch zu verabreichenden Zusammensetzungen enthalten.

Die oral zu verabreichenden Zusammensetzungen werden bevorzugt in Form von Tabletten, Kapseln, Saft, Tropfen, Brausetabletten oder Lyophilisat verabreicht.

Sie können übliche Hilfsstoffe, wie z.B. Lösungsmittel, Lösungsbeschleuniger, Emulgatoren, Lösungsvermittler, Netzmittel, Antischaummittel, Salzbildner, Puffer, Gelbildner, Verdickungsmittel, Filmbildner, Bindemittel, Gleitmittel, Schmiermittel, Formtrennmittel, Fließregulierungsmittel, Zerfallsbeschleuniger oder Sprengmittel, Sorptionsmittel, Füllstoffe sowie Konservierungsmittel, Geschmacks- und Geruchskorrigenzien sowie Färbemittel enthalten.

Bevorzugte Antioxidantien für die orale Verabreichung sind insbesondere Lutein, Lycopen sowie Emblica.

Der Gehalt an Antioxidantien in den oral zu verabreichenden pharmazeutischen Zusammensetzungen beträgt 1-600 mg, bevorzugt 2-300 mg, besonders bevorzugt 5-200 mg.

Die Antioxidantien können in der nach üblichen Verfahren ablaufenden Herstellung von kosmetischen und pharmazeutischen Zusammensetzungen verwendet werden.

Die folgenden Ausführungsbeispiele verdeutlichen die Erfindung, ohne sie zu beschränken.

### Beispiel 1 (Hydrodispersionsgel)

| | |
|---|---|
| Neo Heliopan 1000®(Isoamyl p-Methoxycinnamate) | 5,0 g |
| Neo Heliopan AV®(Ethylhexyl Methoxycinnamate) | 5,0 g |
| Eusolex 6300®(4-Methylbenzylidene Camphor) | 3,0 g |
| Parsol 1789®(Butyl Methoxydibenzoylmethane) | 2,0 g |
| Uvinul T150®(Ehtylhexyl Triazone) | 2,0 g |
| α-Tocopherol | 0,5 g |
| Emblica | 0,5 g |
| Glycerin | 3,0 g |
| Disodium EDTA | 0,1 g |
| Panthenol | 1,0 g |
| E-Wasser | 68,35 g |
| Pemulen TR1®(Acrylates C10-30 Alkyl Acrylate Crosspolymer) | 0,4 g |
| Natriumhydroxid rein | 0,15 g |
| Vergällter Alkohol | 9,0 g |

### Phase A:

4-Methylbenzylidene Camphor, Ethylhexyl Triazone und Butyl Methoxydibenzoylmethane in Isoamyl p-Methoxycinnamate und Ethylhexyl Methoxycinnamate lösen. Tocopherol zugeben, Acrylates C 10-30 Alkyl Acrylate Crosspolymer dispergieren.

### Phase B:

Natriumhydroxid rein in einem Teil E-Wasser lösen.

### Phase C:

Emblica, Glycerin Disodium EDTA und Panthenol in Wasser lösen

### Phase D:

Vergällter Alkohol

Phase C zu Phase A geben, unter Homogenisieren mischen, Phase B zugeben, unter Rühren mischen. Phase D zugeben, unter Rühren mischen.

### Beispiel 2 (Lotion)

| | |
|---|---|
| Tego Care 450®(Polyglyceryl-3 Methylglucose Distearate) | 2,0 g |
| Softisan 100®(Hydrogenated Coco-Glycerides) | 0,5 g |
| Cutina CP®(Cetyl Palmitate) | 0,5 g |
| Lanette C®(Cetyl Alcohol) | 1,5 g |
| Myritol 331®(Cocoglycerides) | 5,0 g |
| Neo Heliopan 1000®(Isoamyl p-Methoxycinnamate) | 5,0 g |
| Uvinul T150 (Ethylhexyl Triazone) | 2,0 g |
| Eusolex 6300®(4-Methylbenzylidene Camphor) | 3,0 g |
| Parsol 1789®(Butylmethoxydibenzoylmethane) | 1,0 g |
| Carbopol 981®(Carbomer) | 0,2 g |
| α-Tocopherolacetat | 1,0 g |
| Lutein | 0,5 g |
| Glycerin | 4,0 g |
| Xanthan Gum | 0,1 g |
| E-Wasser | 64,654g |
| Natriumhydroxid rein | 0,046 g |
| Vergällter Alkohol | 9,0 g |

### Phase A:

Polyglyceryl-3 Methylglucose Distearate, Hydrogenated Coco-Glycerides, Cetyl Palmitate, Cetyl Alcohol und Cocoglycerides erwärmen, darin Ethylhexyl Triazone, 4-Methylbenzylidene Camphor und Butyl Methoxydibenzoylmethane lösen. Tocopherolacetate und Lutein darin lösen.

### Phase B:

Natriumhydroxide in einem Teil Wasser lösen

### Phase C:

Glycerin und E-Wasser mischen, Xanthan Gum und Carbomer darin quellen

### Phase D:

Vergällter Alcohol

Phase C zu Phase A geben, unter Homogenisieren mischen, Phase B zugeben, unter Homogenisieren mischen, Phase D zugeben, unter Homogenisieren mischen.

### Beispiel 3 (Hydrodisperionsgel)

| | |
|---|---|
| Eutanol G®(Octyldodecanol) | 5,0 g |
| Myritol 318®(Carpylic/Capric Triglyceride) | 5,0 g |
| Eusolex 6300®(4-Methylbenzylidene Camphor) | 3,0 g |
| Parsol 1789®(Butyl Methoxydibenzoylmethane) | 1,0 g |
| DL-α-Tocopherol | 0,5 g |
| Pemulen TR1®(Acrylates C10-30 Alkyl Acrylate Crosspolymer) | 0,4 g |
| Panthenol | 1,0 g |
| Glycerin | 3,0 g |
| Disodium EDTA | 0,1 g |
| E-Wasser | 58,85 g |
| Natriumhydroxid rein | 0,15 g |
| Uvinul T150®(Ethylhexyl Triazone) | 2,0 g |
| Vergällter Alkohol | 9,0 g |
| Isopropylmyristat | 5,0 g |
| Neo Heliopan AV (Ethylhexylmethoxycinnamate) | 5,0 g |
| Edelweissextrakt | 1,0 g |

### Phase A:

4-Methylbenzyliden Camphor, Ethylhexyl Triazone und Butyl Methoxydibenzoylmethane in Ethylhexyl Methoxycinnamate und Caprylic/Capric Triglyceride lösen. Octyldodecanol, Isopropylmyristate und Tocopherol zugeben, Acrylates C 10-30 Alkyl Acrylate Crosspolymer dispergieren.

### Phase B:

Natriumhydroxid rein in einem Teil E-Wasser lösen

### Phase C:

Edelweissextrakt, Glycerin Disodium EDTA und Panthenol in Wasser lösen

### Phase D:

Vergällter Alkohol

Phase C zu Phase A geben, unter Homogenisieren mischen, Phase B zugeben, unter Rühren mischen, Phase D zugeben, unter Rühren mischen.

### Beispiel 4 (W/O-Creme)

**Phase A:**

| | |
|---|---|
| Dehymuls PGPH (Polyglyceryl-2 Dipolyhydroxystearate) | 3,0 g |
| Monomuls 90-018 (Glyceryl Oleate) | 2,0 g |
| Myritol 318 (Caprylic/Capric Triglyceride) | 6,0 g |
| Isopropylmyristat | 10,0 g |
| α-Tocopherolacetat | 1,0 g |
| Bienenwachs | 1,0 g |
| Magnesiumstearat | 0,5 g |
| Isoamyl p-Methoxycinnamate | 10,0 g |
| 4-Methylbenzylidene Camphor | 4,0 g |
| Ethylhexyl Triazone | 1,0 g |

Unter Erwärmen lösen

**Phase B:**

| | |
|---|---|
| E-Wasser | 45,8 g |
| Disodium EDTA | 0,1 g |
| Pentylene Glycol | 3,0 g |
| Magnesiumsulfat | 0,5 g |
| α-Bisabolol | 0,1 g |
| Grünteeextrakt | 1,0 g |

Unter Rühren lösen

**Phase C:**

| | |
|---|---|
| Titaniumdioxide | 3,0 g |

**Phase D:**

| | |
|---|---|
| Alcohol | 8,0 g |

Phase B zu Phase A geben, unter Homogenisieren mischen. Unter Homogenisieren Phase C zugeben. Phase D zugeben, unter Homogenisieren mischen.

### Beispiel 5 (Hydrodisperionsgel)

**Phase A:**

| | |
|---|---|
| Neo Heliopan AV (Ethylhexyl Methoxycinnamate) | 2,5 g |
| Neo Heliopan 1000 (Isoamyl p-Methoxycinnamate) | 2,5 g |
| Neo Heliopan 303 (Octocrylene) | 2,5 g |
| α-Tocopherolacetat | 0,25 g |
| Acrylates C 10-30 Alkyl Acrylate Crosspolymer | 0,5 g |

Mischen und dispergieren

**Phase B:**

| | |
|---|---|
| E-Wasser | 30,0 g |
| Neo Heliopan Hydro (Phenylbenzimidazole Sulfonic Acid) | 2,0 g |
| Neo Heliopan AP (Disodium Phenyl Dibenzimidazole Tetrasulfonate) | 2,0 g |
| Natrium Hydroxide rein | 0,7 g |

Lösung herstellen

**Phase C:**

| | |
|---|---|
| E-Wasser | 44,0 g |
| Vergällter Alkohol | 9,5 g |
| Glycerin | 1,5 g |
| Panthenol | 0,5 g |
| Disodium EDTA | 0,05 g |
| | |
| Vitamin C | 0,5 g |
| Emblica | 1,5 g |

Lösung herstellen

Phasen B und C mischen und unter Homogenisieren zu Phase A geben.

### Beispiel 6 (Hartkapsel zur oralen Verabreichung)

| | |
|---|---|
| Lycopen | 8 mg |
| Beta-Carotin | 8 mg |
| Lutein | 8 mg |
| Vitamin C | 50 mg |
| Vitamin E | 25 mg |
| Lactose | 125 mg |
| Magnesiumstearat | 3 mg |
| Aerosil | 3 mg |
| Gelatine | 75 mg aus Kapselhülle |

Die Bestandteile 1-7 werden entsprechend ihrem Mengenverhältnis abgewogen und gesiebt. Danach werden sie in einem Freifallmischer gemischt. Magnesiumstearat wird abgewogen und gesiebt und zu der Hauptmischung aus 1-7 hinzugegeben und gemischt. Die fertige Mischung wird auf einer geeigneten Kapselmaschine in Hart-Kapseln gefüllt.

## Patentansprüche

1. Pharmazeutische Zusammensetzung, umfassend einen wirksamen Gehalt an Antioxidantien zum Schutz der Haut vor Schädigung durch Infrarotstrahlung, wobei die Antioxidantien ausgewählt sind aus der Gruppe bestehend aus N-Acetylcystein, Kaffeesäure, Ester der Kaffeesäure, Emblica, Grünteeextrakt, Edelweissextrakt, Lutein, Lycopen, Traubenkernextrakt, Chicory-Extrakt, Curcumin, Silymarin, Apigenin und Resveratrol und wobei die Schädigung durch Aktivierung der Bildung von Matrixmetalloproteinase-1 hervorgerufen wird.

2. Zusammensetzung nach Anspruch 1 in Form einer topisch zu verabreichenden Zusammensetzung.

3. Zusammensetzung nach Anspruch 2,
**dadurch gekennzeichnet, dass** der Gehalt an Antioxidantien in der Zusammensetzung 0,001-25 Gew.-%, bevorzugt 0,01-10 Gew.-% beträgt.

4. Zusammensetzung nach Anspruch 2 oder 3,
**dadurch gekennzeichnet, dass** die Zusammensetzung des weiteren einen oder mehrere UV-Filter umfasst.

5. Zusammensetzung nach einem der Ansprüche 2 bis 4,
**dadurch gekennzeichnet, dass** die Zusammensetzung des weiteren Hilfsstoffe enthält, die unter Verdickungsmitteln, Glättungsmitteln, anfeuchtenden und/oder feuchthaltenden Substanzen, oberflächenaktiven Mitteln, Konservierungsmitteln, Entschäumern, Fetten, Ölen, Wachsen, Bakteriziden, Parfümen, Treibmitteln, Farbstoffen und/oder Pigmenten ausgewählt sind.

## Claims

1. A pharmaceutical composition comprising an effective amount of antioxidants for protecting the skin from damage by infrared radiation, wherein the antioxidants are selected from the group consisting of N-acetylcysteine, caffeic acid, esters of caffeic acid, emblica, green tea extract, edelweiss extract (*leontopodium-extract),* lutein, lycopene, grape seed extract, chicory extract, curcumin, silymarin, apigenin and resveratrol, and wherein the damage is caused by activation of the formation of matrix metalloproteinase-1.

2. The composition according to claim 1 in the form of a composition to be administered topically.

3. The composition according to claim 2, **characterized in that** the content of antioxidants in the composition is 0.001-25 wt.%, preferably 0.01-10 wt.%.

4. The composition according to claim 2 or 3, **characterised in that** the composition further comprises one or more UV filters.

5. a composition according to any one of claims 2 to 4, **characterized in that** the composition further contains auxiliaries selected from thickening agents, smoothing agents, moistening and/or moisturizing substances, surface-active agents, preservatives, defoamers, greases, oils, waxes, bactericides, perfumes, propellants, dyes and/or pigments.

## Revendications

1. Composition pharmaceutique, comprenant une teneur effective en antioxydants pour protéger la peau contre les dommages provoqués par les rayonnements infrarouges, dans laquelle les antioxydants sont choisis dans le groupe composé de la N-acétylcystéine, l'acide caféique, l'ester d'acide caféique, l'Emblica, l'extrait de thé vert, l'extrait d'edelweiss, la lutéine, le lycopène, l'extrait de pépins de raisin, l'extrait de chicorée, la curcumine, la silymarine, l'apigénine et le resvératrol et dans laquelle les dommages sont provoqués par l'activation de la formation de la métalloprotéase matricielle-1.

2. Composition selon la revendication 1 sous la forme d'une composition à administrer par voie topique.

3. Composition selon la revendication 2,
**caractérisée en ce que** la teneur en antioxydants dans la composition représente de 0,001 à 25 % en poids, de préférence de 0,01 à 10 % en poids.

4. Composition selon la revendication 2 ou 3,
**caractérisée en ce que** la composition comprend en outre un ou plusieurs filtres UV.

5. Composition selon l'une des revendications 2 à 4,
**caractérisée en ce que** la composition contient en outre des excipients qui sont choisis parmi des agents épaississants, des agents lissants, des substances humidifiantes et/ou avec une teneur en humidité, des agents de surface surfactants, des agents conservateurs, des agents anti-mousse, des graisses, des huiles, des cires, des bactéricides, des parfums, des agents gonflants, des colorants et/ou des pigments.
